(19) Europäisches Patentamt
European Patent Office
Office européen des brevets

(11) **EP 4 641 580 A1**

(12) **EUROPEAN PATENT APPLICATION**

(43) Date of publication:
**29.10.2025 Bulletin 2025/44**

(21) Application number: **24465518.9**

(22) Date of filing: **26.04.2024**

(51) International Patent Classification (IPC):
**G16H 20/40** (2018.01)    **G16H 50/30** (2018.01)
**G16H 30/40** (2018.01)    **G16H 50/20** (2018.01)
**G16H 50/50** (2018.01)    **G16H 50/70** (2018.01)

(52) Cooperative Patent Classification (CPC):
**G16H 50/20; G16H 20/40; G16H 30/40;**
**G16H 50/30; G16H 50/50; G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **Sharma, Puneet**
  **Princeton Junction, 08550 (US)**
• **Gulsun, Mehmet Akif**
  **Princeton, 08540 (US)**
• **Itu, Lucian Mihai**
  **500294 Brasov (RO)**

(74) Representative: **HKW Intellectual Property PartG mbB**
**Theresienhöhe 12**
**80339 München (DE)**

(54) **AI BASED RISK ASSESSMENT OF MEDICAL PROCEDURES**

(57) Systems and methods for clinical decision support are provided. One or more input medical images of an anatomical object of a patient are received. Characteristics of the anatomical object are extracted from the one or more input medical images. A risk associated with a medical procedure to be performed on the anatomical object is determined based on the one or more input medical images and the extracted characteristics of the anatomical object using one or more machine learning based risk assessment models. A simulation of the medical procedure is performed on the anatomical object based on the one or more input medical images, the extracted characteristics of the anatomical object, and the risk associated with the medical procedure. One or more clinical decisions associated with the medical procedure are automatically made based on the risk associated with the medical procedure and results of the simulation. The one or more clinical decisions are output.

**EP 4 641 580 A1**

**Description**

TECHNICAL FIELD

The present invention relates generally to AI (artificial intelligence) based risk assessment of medical procedures, and in particular to AI based risk assessment of stent under-expansion for clinical decision support.

BACKGROUND

Risk assessment associated with a medical procedure is important for providing clinical decision support. For example, PCI (percutaneous coronary intervention) is a medical procedure often performed as a treatment for CAD (coronary artery disease). CAD is a cardiovascular disease that occurs when plaque builds up in the arteries that supply blood to the heart, causing them to narrow and restrict blood flow. The narrowing of the arteries is referred to as a stenosis. PCI may be performed to relieve symptoms such as angina, improve blood flow to the heart, and reduce the risk of heart attack. During PCI, a stent may be placed at the site of a stenosis to keep the artery open. To place the stent, the stent is initially crimped onto a deflated balloon at the tip of a catheter. Once the catheter reaches the stenosis, the balloon is inflated, expanding the stent. This expansion forces the plaque buildup against the artery wall, widening the artery and restoring blood flow.

One risk associated with PCI is stent under-expansion. Proper expansion of the stent is important for ensuring optimal stent apposition to the arterial wall and minimizing the risk of complications such as stent thrombosis or restenosis. Conventionally, invasive intra-coronary imaging techniques using IVUS (intravascular ultrasound) and OCT (optical coherence tomography) have been proposed to assess the risk of stent under-expansion. However, IVUS/OCT imaging increases the duration and cost of the procedure and carries additional risk to the patient.

BRIEF SUMMARY OF THE INVENTION

In accordance with one or more embodiments, systems and methods for clinical decision support are provided. One or more input medical images of an anatomical object of a patient are received. Characteristics of the anatomical object are extracted from the one or more input medical images. A risk associated with a medical procedure to be performed on the anatomical object is determined based on the one or more input medical images and the extracted characteristics of the anatomical object using one or more machine learning based risk assessment models. A simulation of the medical procedure is performed on the anatomical object based on the one or more input medical images, the extracted characteristics of the anatomical object, and the risk associated with the medical procedure. One or more clinical decisions associated with the medical procedure are automatically made based on the risk associated with the medical procedure and results of the simulation. The one or more clinical decisions are output.

In one embodiment, the one or more clinical decisions comprises a decision to perform the medical procedure. One or more preoperative medical images of the anatomical object of the patient for performing the medical procedure are received. Additional characteristics of the anatomical object are extracted from the one or more preoperative medical images. An updated risk associated with the medical procedure to be performed on the anatomical object is determined based on the one or more preoperative medical images and the extracted additional characteristics of the anatomical object using the one or more machine learning based risk assessment models. An additional simulation of the medical procedure is performed on the anatomical object based on the one or more preoperative medical images, the extracted additional characteristics of the anatomical object, and the updated risk associated with the medical procedure. One or more additional clinical decisions associated with the medical procedure are automatically made based on the updated risk associated with the medical procedure and results of the additional simulation. The one or more additional clinical decisions are output.

In one embodiment, the medical procedure is performed based on the one or more additional clinical decisions. A post-procedure risk associated with the performed medical procedure may be determined.

In one embodiment, the steps of 1) determining the risk associated with the medical procedure based on the results of the simulation and 2) performing the simulation of the medical procedure are iteratively repeated to optimize a cost function.

In one embodiment, the anatomical object comprises a stenosis. Geometry characteristics associated with the stenosis and plaque characteristics associated with the stenosis are extracted from the one or more input medical images.

In one embodiment, characteristics of the medical procedure are received. The simulation of the medical procedure is performed on the anatomical object further based on the characteristics of the medical procedure.

In one embodiment, the anatomical object comprises a stenosis, the medical procedure comprises a PCI (percutaneous coronary intervention) procedure to place a stent at the stenosis, and the risk comprises a risk of under-expansion of the stent.

In one embodiment, the one or more input medical images comprises at least one of a photon counting computed tomography image, an x-ray angiography image, an intravascular ultrasound image, or an optical coherence tomography image.

These and other advantages of the invention will be apparent to those of ordinary skill in the art by reference to the following detailed description and the accompanying drawings.

BRIEF DESCRIPTION OF THE DRAWINGS

Figure 1 shows a workflow for minimizing risk of stent under-expansion, in accordance with one or more embodiments;
Figure 2 shows a method for clinical decision support, in accordance with one or more embodiments;
Figure 3 shows a method for updated clinical decision support, in accordance with one or more embodiments;
Figure 4 shows an exemplary artificial neural network that may be used to implement one or more embodiments;
Figure 5 shows a convolutional neural network that may be used to implement one or more embodiments;
Figure 6 shows a data flow diagram according to an embodiment for using a generative adversarial network, in accordance with one or more embodiments;
Figure 7 shows a schematic structure of a recurrent machine learning model that may be used to implement one or more embodiments; and
Figure 8 shows a high-level block diagram of a computer that may be used to implement one or more embodiments.

DETAILED DESCRIPTION

The present invention generally relates to methods and systems for AI based risk assessment of medical procedures for clinical decision support. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa.

Risk assessment associated with medical procedures is important for clinical decision support. For example, a PCI procedure may be performed to place a stent at a stenosis for treating CAD. Conventional approaches for risk assessment of stent under-extension involve invasive intra-coronary imaging techniques, which result in increased duration and cost of the procedure and additional risk to the patient. Embodiments described herein provide for a non-invasive approach for AI based risk assessment of stent under-expansion. Such AI based risk assessment may be performed prior to the PCI procedure placing the stent, thereby enabling tailoring of the PCI procedure on a patient-specific basis and resulting in better patient outcomes, reduced cost, reduced procedure time, and reduced patient risk.

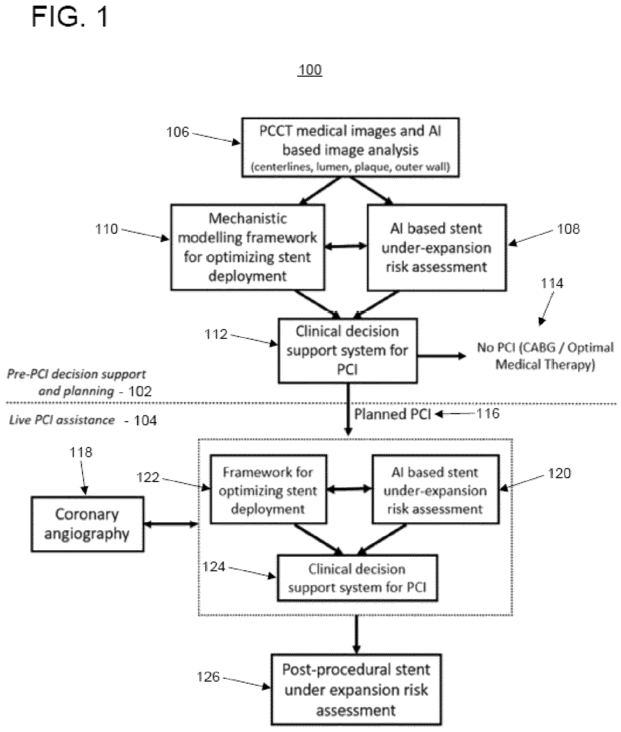

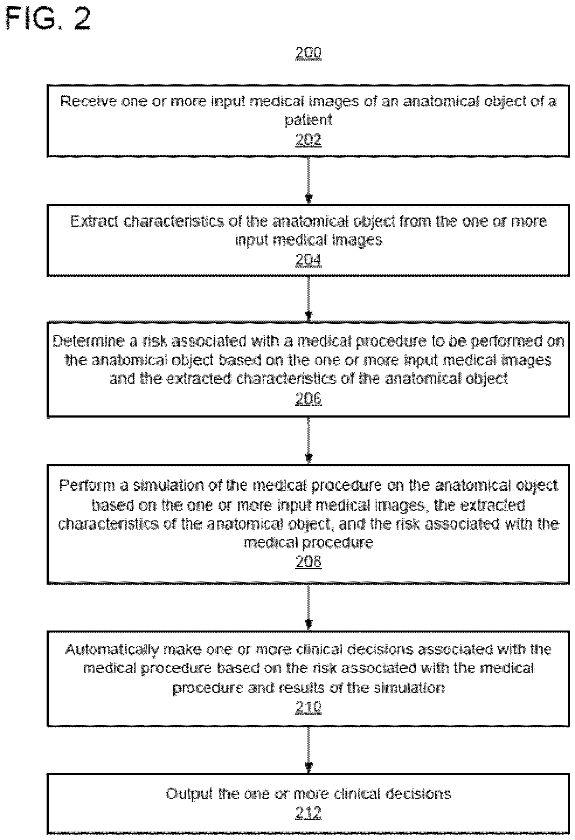

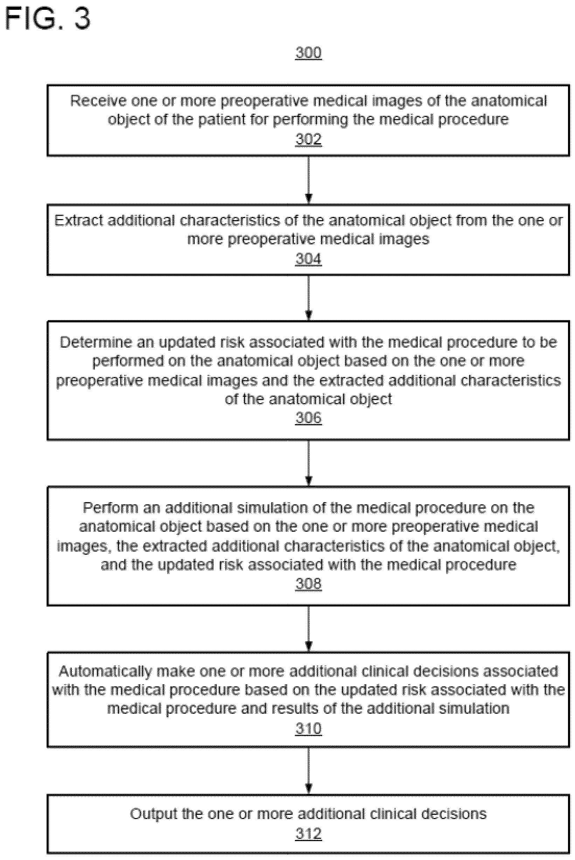

Figure 1 shows a workflow 100 for minimizing risk of stent under-expansion, in accordance with one or more embodiments. Workflow 100 comprises a stage 102 for pre-PCI decision support and planning and a stage 104 for live PCI assistance. Stage 102 for pre-PCI decision support and planning is performed prior to performing the PCI procedure placing a stent and stage 104 for live PCI assistance is performed during and after performing the PCI procedure placing the stent. Figure 1 will be described together with Figure 2 and Figure 3. Method 200 of Figure 2 is performed during stage 102 for pre-PCI decision support and planning. Method 300 of Figure 3 is performed during stage 104 for live PCI assistance.

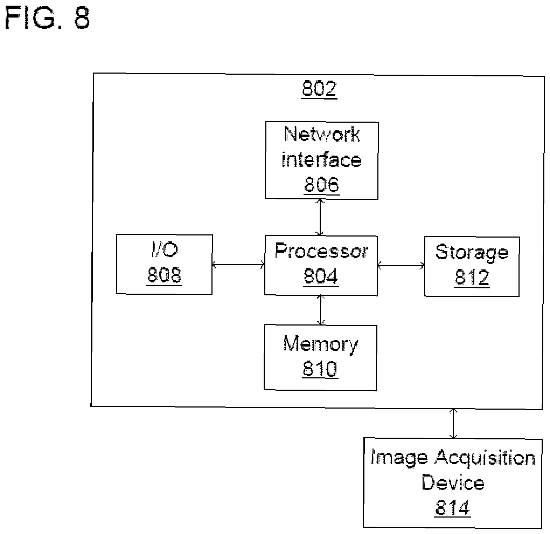

Figure 2 shows a method 200 for clinical decision support, in accordance with one or more embodiments. The steps and sub-steps of method 200 may be performed by one or more suitable computing devices, such as, e.g., computer 802 of Figure 8.

At step 202 of Figure 2, one or more input medical images of an anatomical object of a patient are received. In one example, as shown in workflow 100 of Figure 1, the one or more input medical images are PCCT (photon-counting computed tomography) medical images received at step 106. In one embodiment, the anatomical object is a stenosis (i.e., a narrowing or blockage of a vessel) of the patient. However, the anatomical object may be any other suitable anatomical object of interest of the patient, such as, e.g., organs, vessels, bones, tumors or other abnormalities, etc.

In one embodiment, the one or more input medical images comprise PCCT images. PCCT imaging utilizes photon-counting detectors, which can distinguish individual photons and measure their energy levels. As compared with traditional CT (computed tomography), PCCT provides improved spatial resolution, reduced radiation dose, and better material decomposition capabilities. In one embodiment, the one or more input medical images comprise CT images. However, the one or more input medical images may comprise images of any other suitable modality, such as, e.g., MRI (magnetic resonance imaging), US (ultrasound), x-ray, or any other medical imaging modality or combinations of medical imaging modalities. The one or more input medical images may comprise 2D (two dimensional) images and/or 3D (three dimensional) volumes, and may comprise a single image or a plurality of images.

In one embodiment, optionally, medical procedure characteristics and/or patient characteristics are also received at step 202 of Figure 2. For instance, where the medical procedure is PCI, the medical procedure characteristics may comprise, e.g., specific characteristics of the stent delivery (e.g., location of deployment, type of balloon, inflation pressure, pre- and post-stenting dilation, the proximal and/or distal landing point, etc.), stent properties (e.g., length, size, material properties, type (e.g., open cell or closed cell), maximum diameter at a given pressure, etc.). The patient characteristics may comprise, e.g., demographics, medical records, diagnoses, treatments, test/lab results, or any other characteristic of the patient.

The one or more input medical images (and optionally the medical procedure characteristics and/or patient characteristics) may be received, for example, by directly receiving the one or more input medical images from the image acquisition device (e.g., image acquisition device 814 of Figure 8) as the images are acquired, by loading the one or more input medical images (and the medical procedure characteristics and/or patient characteristics) from a storage or memory of a computer system (e.g., storage 812 or memory 810 of computer 802 of Figure 8), or by receiving the one or more input medical images (and the medical procedure characteristics and/or patient characteristics) from a remote computer system (e.g., computer 802 of Figure 8). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 204 of Figure 2, characteristics of the anatomical object are extracted from the one or more input medical images. In one example, as shown in workflow 100 of Figure 1, the characteristics of the anatomical object are extracted according to an AI based image analysis at step 106.

The characteristics of the anatomical object may comprise any suitable characteristics of the anatomical object. In one embodiment, where the anatomical object is a stenosis, the characteristics may comprise geometry characteristics associated with the stenosis and plaque characteristics associated with the stenosis. The geometry characteristics may comprise anatomical and morphological characteristics, such as, e.g., identification of centerlines of the vessel, identification and assessment of the lumen, identification of the outerwall, etc. PCCT images allow for a more precise assessment of the geometry characteristics given the high-resolution 3D image data inside and around the stenosis, avoiding artifacts such as blooming. The plaque characteristics may comprise, e.g., location, volume/amount, type (e.g., calcified, fibrotic, fibro-fatty, or nodular), extent, etc. The multi-energy capabilities of PCCT, along with its high spatial resolution, provide for better characterization of tissue and material composition. In one embodiment, the plaque characteristics may comprise results of an assessment of pericoronary adipose tissue performed using PCCT images.

In one embodiment, optionally, the characteristics of the anatomical object may comprise features extracted from a radiomic analysis of the one or more input medical images. For example, the radiomic analysis may be performed by overlaying markers on the one or more input medical images to identify a start and end of a stenosis, identifying a region of interest around the lesion based on the markers, and extracting radiomic GLCM (gray level co-occurrence matrix) features from the one or more input medical images within the region of interest.

The characteristics of the anatomical object may be extracted using one or more machine learning based feature extraction networks. The feature extraction networks may be implemented using any suitable (e.g., well-known) machine learning based network. The feature extraction networks receive as input the one or more input medical images and generates as output the characteristics of the anatomical object. The feature extraction networks are trained during a prior offline or training stage using training data according to any suitable (e.g., well-known) approach. Once trained, the feature extraction networks are applied during an online or training stage, e.g., to perform step 204 of Figure 2.

At step 206 of Figure 2, a risk associated with a medical procedure to be performed on the anatomical object is determined based on the one or more input medical images and the extracted characteristics of the anatomical object. In one example, as shown in workflow 100 of Figure 1, the risk is determined at AI based stent under-expansion risk assessment step 108.

In one embodiment, the medical procedure is a PCI procedure to place a stent at a stenosis and the risk associated with the PCI comprises a risk of under-expansion of the stent. However, the medical procedure and the risk associated with the medical procedure may comprise any suitable medical procedure and/or any suitable risk. For example, the risk may comprise the risk of side branch closure.

In one embodiment, the risk is represented as a risk score. However, the risk may also be represented as a binary risk/no risk, a probability of risk, a classification of risk into a plurality of categories, or in any other suitable manner. The risk is determined using one or more machine learning based risk assessment models. The risk assessment models may be implemented using any suitable (e.g., well-known) machine learning based risk assessment models. The risk assessment models receive as input the one or more input medical images, the extracted characteristics of the anatomical object, and optionally the patient characteristics and generates as output the risk associated with the medical procedure.

The risk assessment models are trained during a prior offline or training stage using training data according to any suitable (e.g., well-known) approach. The training data may comprise training images labelled to identify observed stent under-expansion, stent thrombus, and in-stent restenosis. The observed stent under-expansion may be, e.g., defined at a frame level or lesion, defined automatically based on post-PCI lumen diameter or area (e.g., as determined from a coronary angiography or IVUS/OCT imaging), and/or defined by a user (e.g., experts). The labels may be assessed during or immediately after the PCI procedure or may be assessed in the long term (e.g., for an in-stent restenosis). In one embodiment, the training data may comprise synthetic training images displaying rare anatomical configurations. The synthetic training images may be generated, for example, using a GAN (generative adversarial network). The synthetic training images may be used for pre-training the risk assessment models. Once trained, the risk assessment models are applied during an online or training stage, e.g., to perform step 206 of Figure 2.

At step 208 of Figure 2, a simulation of the medical procedure is performed on the anatomical object based on the one or more input medical images, the extracted characteristics of the anatomical object, and the risk associated with the medical procedure. For example, the simulation may be of performing a PCI at a stenosis. In one example, the simulation of the medical procedure is performed via a mechanistic modelling framework for optimizing a stent deployment at step 110.

In one embodiment, the simulation is performed using one or more mechanistic models. Mechanistic models are mathematical models or equations based on natural laws (e.g., biological, physical, and/or chemical laws). The mechanistic models receive as input the one or more input medical images, the extracted characteristics of the anatomical object, the risk associated with the medical procedure, and optionally the medical procedure characteristics and generates as output results of the simulation of the medical procedure.

In some embodiments, the simulated medical procedure additionally or alternatively comprises passing the stent through the stenosis. If passing through the stent through the stenosis is difficult, pre-dilation and/or atherectomy may be performed.

In one embodiment, an iterative workflow is provided by iteratively repeating steps 206 and 208 to optimize (e.g., minimize) a cost function. Step 206 is iteratively repeated using results of the simulation of the medical procedure (determined at step 208) and step 208 is iteratively repeated using the risk associated with the medical procedure (determined at step 206). The iterative workflow is provided by varying parameters of the medical procedure. For example, where the medical procedure is performing a PCI placing a stent at a stenosis, the parameters may comprise different locations of stent deployment, different types of stents (e.g., size, length, material properties, etc.), varying the number of stents, different procedural settings (e.g., inflation pressure), pre- and post-stenting dilation, etc.

The cost function may be defined based on one or more measures of interest (e.g., lumen size after stent deployment, stent apposition, etc.), which is optimized as part of the iterative workflow. The cost function may be further defined based on one or more of: the risk associated with the medical procedure (e.g., the risk of stent under-expansion and the risk of side branch closure), the extent of edge dissections and perforation, post-stent deployment hemodynamic characteristics (e.g., using a hemodynamic model to estimated post-PCT FFR (fractional flow reserve) or using a machine learning based model to predict post-PCI FFR values given an anatomical model after stent deployment).

In one embodiment, the iterative workflow is further extended by considering a mechanistic modeling of atherectomy. Thus, if, within the initial iteration, no satisfactory risk is obtained, atherectomy may be considered and simulated to modify the anatomical characteristics of the coronary artery and thus improve the risk (e.g., of stent under-expansion). Hence, one of the outputs may be an indication for atherectomy prior to the medical procedure.

At step 210 of Figure 2, one or more clinical decisions associated with the medical procedure are automatically made based on the risk associated with the medical procedure and results of the simulation. The one or more clinical decisions may be made using any suitable (e.g., well-known) approach based on the risk associated with the medical procedure and results of the simulation. The one or more clinical decisions may be whether to perform the medical procedure. In one example, as shown in workflow 100 of Figure 1, clinical decision support system for PCI 112 determines whether to perform the planned PCI 116 or no PCI 114 (and perform CAGB (coronary artery bypass surgery) or optimal medical therapy instead).

In one embodiment, the one or more clinical decisions also comprises characteristics of the medical procedure, such as, e.g., deploy locations of one or more stents, stent characteristics (length, size, type (e.g., open cell for bifurcations or closed cell for ostial lesions), inflation pressure during pre-dilation, stent deployment, and post-dilation, requirement for pre- and post-stenting dilation, requirement for atherectomy, etc.

In one embodiment, virtual angiographies may also be generated from the one or more input medical images (e.g., PCCT images), covering both the pre- and post-stenting scenarios, to support clinicians in understanding the suggested clinical decisions for taking the best course of action.

At step 212 of Figure 2, the one or more clinical decisions are output. For example, the one or more clinical decisions can be output by displaying the one or more clinical decisions on a display device of a computer system (e.g., I/O 808 of computer 802 of Figure 8), storing the one or more clinical decisions on a memory or storage of a computer system (e.g., memory 810 or storage 812 of computer 802 of Figure 8), or by transmitting the one or more clinical decisions to a remote computer system (e.g., computer 802 of Figure 8).

In one embodiment, for example, where the one or more clinical decisions comprise a decision to perform the medical procedure, one or more preoperative medical images of the patient are acquired for performing the medical procedure. Method 200 of Figure 2 are repeated as method 300 of Figure 3 by applying the same networks/models but using the one or more preoperative medical images as the one or more input medical images for making additional clinical decisions. For example, if the one or more clinical decisions comprises a decision to schedule the patient for a coronary angiography and PCI, steps 206 and 208 may be iterated to update the risk and the simulation using information extracted from CT images acquired during the coronary angiographies. Such CT images may provide additional insight since: 1) the coronary angiography allows for a more precise analysis of the lumen information and certain changes in the anatomy may occur between the time of the acquisition of the one or more input medical images (e.g., PCCT images) and the coronary angiography images; and 2) patient characteristics may change over time.

Since one or more machine learning based models are utilized for determining the risk (at step 206 of Figure 2), the updated risk may be determined in substantially real time. However, the mechanistic modeling may involve longer runtimes and may not be suited to performing simulations in substantially real time during the medical procedure. Accordingly, in one embodiment, the mechanistic modeling may be substituted with a physics informed machine learning based simulation network, which has the same inputs and outputs of the mechanistic model.

Figure 3 shows a method 300 for updated clinical decision support, in accordance with one or more embodiments. The steps and sub-steps of method 300 may be performed by one or more suitable computing devices, such as, e.g., computer 802 of Figure 8.

At step 302 of Figure 3, one or more preoperative medical images of the patient for performing the medical procedure are received. In one embodiment, the one or more preoperative medical images are x-ray images, IVUS images, and/or OCT images acquired during a coronary angiography for guidance in performing a PCI. For example, as shown in workflow 100 of Figure 1, the one or more preoperative medical images may be acquired during coronary angiography 118. However, the one or more preoperative medical images may be of any other suitable modality or modalities. The one or more preoperative medical images may comprise 2D images and/or 3D volumes, and may comprise a single image or a plurality of images.

The one or more preoperative medical images may be received, for example, by directly receiving the one or more preoperative medical images from the image acquisition device (e.g., image acquisition device 814 of Figure 8) as the images are acquired, by loading the one or more preoperative medical images from a storage or memory of a computer system (e.g., storage 812 or memory 810 of computer 802 of Figure 8), or by receiving the one or more preoperative medical images from a remote computer system (e.g., computer 802 of Figure 8). Such a computer system or remote computer system may comprise one or more patient databases.

At step 304 of Figure 3, additional characteristics of the anatomical object are extracted from the one or more preoperative medical images. The additional characteristics of the anatomical object may comprise any suitable characteristics of the anatomical object. The additional characteristics may be extracted from the one or more preoperative medical images using the one or more machine learning based feature extraction networks, as described above at step 204 of Figure 2.

At step 306 of Figure 3, an updated risk associated with the medical procedure to be performed on the anatomical object is determined based on the one or more preoperative medical images and the extracted additional characteristics of the anatomical object. In one example, as shown in workflow 100 of Figure 1, the updated risk is determined at AI based stent under-expansion risk assessment step 120. The updated risk is similar to the risk determined at step 206 of Figure 2. The updated risk may be determined using the one or more machine learning based risk assessment models, as described above at step 206 of Figure 2.

At step 308 of Figure 3, an additional simulation of the medical procedure is performed on the anatomical object based on the one or more preoperative medical images, the extracted additional characteristics of the anatomical object, and the updated risk associated with the medical procedure. In one example, the additional simulation of the medical procedure is performed via the framework for optimizing stent deployment at step 122. The additional simulation may be performed using one or more machine learning based simulation models, which has the same inputs and outputs as the one or more mechanistic models described above at step 208 of Figure 2. The one or more machine learning based simulation models may be trained ruing a prior offline or training stage using training data. The training data may comprise synthetic data and outputs computed by the one or more mechanistic models, and thus, the one or more machine learning based simulation models may be surrogate models of the one or more mechanistic models.

In one embodiment, steps 306 and 308 are iteratively repeated to optimize a cost function, as described above at steps 206 and 208 of Figure 2.

At step 310 of Figure 3, one or more additional clinical decisions associated with the medical procedure are automatically made based on the updated risk associated with the medical procedure and results of the additional simulation. The one or more additional clinical decisions may be made using any suitable (e.g., well-known) approach based on the updated risk associated with the medical procedure and results of the additional simulation. The one or more additional clinical decisions may be similar to the one or more clinical decisions described above at step 210 of Figure 2. In one example, as shown in workflow 100 of Figure 1, the one or more additional clinical decisions may be automatically made by clinical decision support system for PCI 124 determines.

At step 312 of Figure 3, the one or more additional clinical decisions are output. For example, the one or more additional clinical decisions can be output by displaying the one or more additional clinical decisions on a display device of a computer system (e.g., I/O 808 of computer 802 of Figure 8), storing the one or more additional clinical decisions on a memory or storage of a computer system (e.g., memory 810 or storage 812 of computer 802 of Figure 8), or by transmitting the one or more additional clinical decisions to a remote computer system (e.g., computer 802 of Figure 8).

In one embodiment, the medical procedure (e.g., PCI) may be performed based on the one or more additional clinical decisions.

In one embodiment, a post-procedure risk associated with the medical procedure may be determined using one or more additional risk assessment models. For example, where the medical procedure is PCI with stent placement, the post-procedure risk may comprise risks resulting from stent under-expansions, such as, e.g., in-stent thrombus or lesion re-occlusion. In-stent thrombus refers to the formation of a blood clot or thrombus within the stent after its placement in the coronary artery, and can lead to vessel occlusion, impaired blood flow, and adverse clinical outcomes such as myocardial infarction, stent thrombosis, and death. The risk of in-stent thrombus can be minimized by optimizing stent placement, ensuring adequate lesion preparation, use of antiplatelet and anticoagulant medications, and closely monitoring of the patients. The post-procedure risk may be determined using one or more machine learning based additional risk assessment models. The additional risk assessment models may be implemented using any suitable (e.g., well-known) machine learning based models. The machine learning based additional risk assessment models receive as input medical images (e.g., x-ray angiography, OCT, IVUS) of the patient acquired after the medical procedure is performed. For example, such medical images may be acquired immediately after placement of the stent or by follow-up CT, e.g., performed one year after placement of the stent. Additionally, the machine learning based risk assessment models may also receive as input blood tests of the patient after a certain period of time after the medical procedure is performed indicating inflammation in the body.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the machine learning based models/networks utilized at steps 106, 108, 120, and 126 of Figure 1, the machine learning based models/networks utilized at steps 204 and 206 of Figure 2, and the machine learning based models/networks utilized at steps 304 and 306 of Figure 3, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

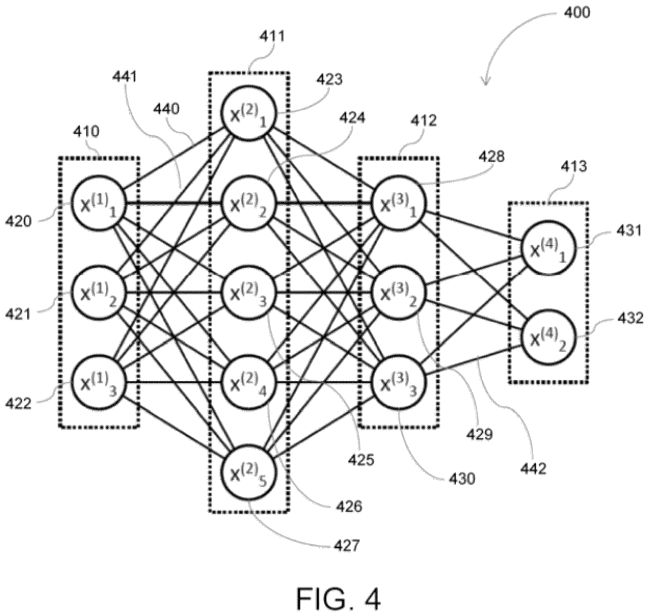

Figure 4 shows an embodiment of an artificial neural network 400 that may be used to implement one or more machine learning models described herein. Alternative terms for "artificial neural network" are "neural network", "artificial neural net" or "neural net".

The artificial neural network 400 comprises nodes 420, ..., 432 and edges 440, ..., 442, wherein each edge 440, ..., 442 is a directed connection from a first node 420, ..., 432 to a second node 420, ..., 432. In general, the first node 420, ..., 432 and the second node 420, ..., 432 are different nodes 420, ..., 432, it is also possible that the first node 420, ..., 432 and the second node 420, ..., 432 are identical. For example, in Figure 4 the edge 440 is a directed connection from the node 420 to the node 423, and the edge 442 is a directed connection from the node 430 to the node 432. An edge 440, ..., 442 from a first node 420, ..., 432 to a second node 420, ..., 432 is also denoted as "ingoing edge" for the second node 420, ..., 432 and as "outgoing edge" for the first node 420, ..., 432.

In this embodiment, the nodes 420, ..., 432 of the artificial neural network 400 can be arranged in layers 410, ..., 413, wherein the layers can comprise an intrinsic order introduced by the edges 440, ..., 442 between the nodes 420, ..., 432. In particular, edges 440, ..., 442 can exist only between neighboring layers of nodes. In the displayed embodiment, there is an input layer 410 comprising only nodes 420, ..., 422 without an incoming edge, an output layer 413 comprising only nodes 431, 432 without outgoing edges, and hidden layers 411, 412 in-between the input layer 410 and the output layer 413. In general, the number of hidden layers 411, 412 can be chosen arbitrarily. The number of nodes 420, ..., 422 within the input layer 410 usually relates to the number of input values of the neural network, and the number of nodes 431, 432 within the output layer 413 usually relates to the number of output values of the neural network.

In particular, a (real) number can be assigned as a value to every node 420, ..., 432 of the neural network 400. Here, $x^{(n)}_i$ denotes the value of the i-th node 420, ..., 432 of the n-th layer 410, ..., 413. The values of the nodes 420, ..., 422 of the input layer 410 are equivalent to the input values of the neural network 400, the values of the nodes 431, 432 of the output layer 413 are equivalent to the output value of the neural network 400. Furthermore, each edge 440, ..., 442 can comprise a weight being a real number, in particular, the weight is a real number within the interval [-1, 1] or within the interval [0, 1]. Here, $w^{(m,n)}_{i,j}$ denotes the weight of the edge between the i-th node 420, ..., 432 of the m-th layer 410, ..., 413 and the j-th node 420, ..., 432 of the n-th layer 410, ..., 413. Furthermore, the abbreviation $w^{(n)}_{i,j}$ is defined for the weight $w^{(n,n+1)}_{i,j}$.

In particular, to calculate the output values of the neural network 400, the input values are propagated through the neural network. In particular, the values of the nodes 420, ..., 432 of the (n+1)-th layer 410, ..., 413 can be calculated based on the values of the nodes 420, ..., 432 of the n-th layer 410, ..., 413 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 410 are given by the input of the neural network 400, wherein values of the first hid-den layer 411 can be calculated based on the values of the input layer 410 of the neural network, wherein values of the second hidden layer 412 can be calculated based in the values of the first hidden layer 411, etc.

In order to set the values $w^{(m,n)}_{i,j}$ for the edges, the neural network 400 has to be trained using training data. In particular, training data comprises training input data and training output data (denoted as $t_j$). For a training step, the neural network 400 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 400 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta^{(n)_j} \;=\; \left(x^{(n+1)_j} - t^{(n+1)_j}\right)\cdot f'\left(x^{(n)_i}\cdot w^{(n)_{i,j}}\right)$$

if the (n+1)-th layer is the output layer 413, wherein f' is the first derivative of the activation function, and $t^{(n+1)}_j$ is the comparison training value for the j-th node of the output layer 413.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / image, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

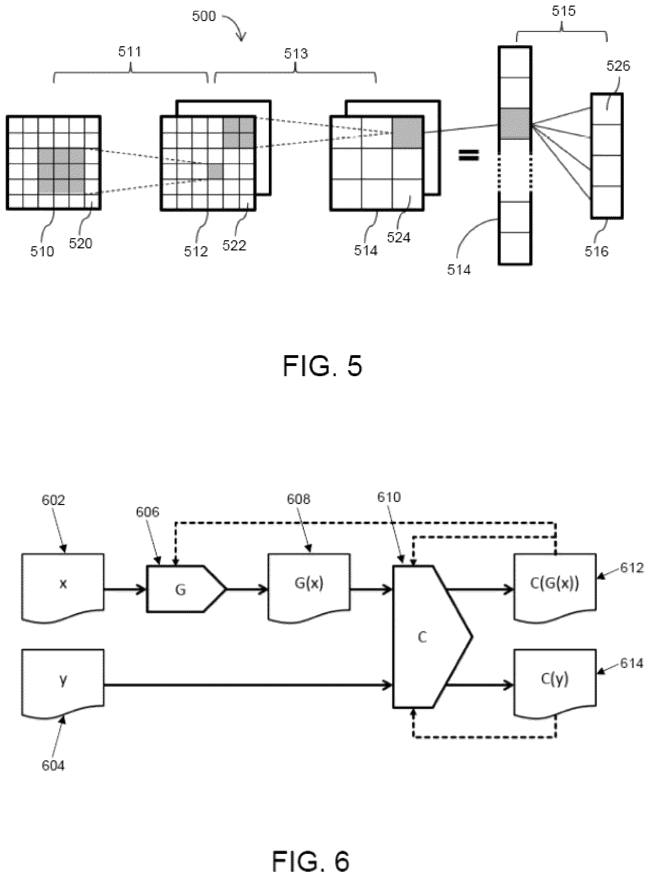

Figure 5 shows an embodiment of a convolutional neural network 500 that may be used to implement one or more machine learning models described herein. In the displayed embodiment, the convolutional neural network comprises 500 an input node layer 510, a convolutional layer 511, a pooling layer 513, a fully connected layer 514 and an output node layer 516, as well as hidden node layers 512, 514. Alternatively, the convolutional neural network 500 can comprise several convolutional layers 511, several pooling layers 513 and several fully connected layers 515, as well as other types of layers. The order of the layers can be chosen arbitrarily, usually fully connected layers 515 are used as the last layers before the output layer 516.

In particular, within a convolutional neural network 500 nodes 520, 522, 524 of a node layer 510, 512, 514 can be considered to be arranged as a d-dimensional matrix or as a d-dimensional image. In particular, in the two-dimensional case the value of the node 520, 522, 524 indexed with i and j in the n-th node layer 510, 512, 514 can be denoted as x(n)[i, j]. However, the arrangement of the nodes 520, 522, 524 of one node layer 510, 512, 514 does not have an effect on the calculations executed within the convolutional neural network 500 as such, since these are given solely by the structure and the weights of the edges.

A convolutional layer 511 is a connection layer between an anterior node layer 510 (with node values x(n-1)) and a posterior node layer 512 (with node values x(n)). In particular, a convolutional layer 511 is characterized by the structure and the weights of the incoming edges forming a convolution operation based on a certain number of kernels. In particular, the structure and the weights of the edges of the convolutional layer 511 are chosen such that the values x(n) of the nodes 522 of the posterior node layer 512 are calculated as a convolution x(n) = K * x(n-1) based on the values x(n-1) of the nodes 520 anterior node layer 510, where the convolution * is defined in the two-dimensional case as

$$x_k^{(n)}[i,j] = \left(K \ast x^{(n-1)}\right)[i,j] = \sum_{i'}\sum_{j'} K[i',j']\cdot x^{(n-1)}[i-i',\, j-j'].$$

Here the kernel K is a d-dimensional matrix (in this embodiment, a two-dimensional matrix), which is usually small compared to the number of nodes 520, 522 (e.g., a 3x3 matrix, or a 5x5 matrix). In particular, this implies that the weights of the edges in the convolution layer 511 are not independent, but chosen such that they produce said convolution equation. In particular, for a kernel being a 3x3 matrix, there are only 9 independent weights (each entry of the kernel matrix corresponding to one independent weight), irrespectively of the number of nodes 520, 522 in the anterior node layer 510 and the posterior node layer 512.

In general, convolutional neural networks 500 use node layers 510, 512, 514 with a plurality of channels, in particular, due to the use of a plurality of kernels in convolutional layers 511. In those cases, the node layers can be considered as (d+1)-dimensional matrices (the first dimension indexing the channels). The action of a convolutional layer 511 is then a two-dimensional example defined as

$$x^{(n)_b}[i,j] = \sum_a K_{a,b} \ast x^{(n-1)_a}[i,j] = \sum_a \sum_{i'} \sum_{j'} K_{a,b}[i',j']\cdot x^{(n-1)_a}[i-i',j-j']$$

where $x^{(n-1)_a}$ corresponds to the a-th channel of the anterior node layer 510, $x^{(n)_b}$ corresponds to the b-th channel of the posterior node layer 512 and $K_{a,b}$ corresponds to one of the kernels. If a convolutional layer 511 acts on an anterior node layer 510 with A channels and outputs a posterior node layer 512 with B channels, there are A B independent d-dimensional kernels $K_{a,b}$.

In general, in convolutional neural networks 500 activation functions are used. In this embodiment re ReLU (acronym for "Rectified Linear Units") is used, with R(z) = max(0, z), so that the action of the convolutional layer 511 in the two-dimensional example is

$$x^{(n)_b}[i,j] = R\left(\sum_a \left(K_{a,b} * x^{(n-1)a}\right)[i,j]\right) = R\left(\sum_a \sum_{i'} \sum_{j'} K_{a,b}[i',j'] \cdot x^{(n-1)a}[i-i', j-j']\right)$$

It is also possible to use other activation functions, e.g., ELU (acronym for "Exponential Linear Unit"), LeakyReLU, Sigmoid, Tanh or Softmax.

In the displayed embodiment, the input layer 510 comprises 36 nodes 520, arranged as a two-dimensional 6x6 matrix. The first hidden node layer 512 comprises 72 nodes 522, arranged as two two-dimensional 6x6 matrices, each of the two matrices being the result of a convolution of the values of the input layer with a 3x3 kernel within the convolutional layer 511. Equivalently, the nodes 522 of the first hidden node layer 512 can be interpreted as arranged as a three-dimensional 2x6x6 matrix, wherein the first dimension correspond to the channel dimension.

The advantage of using convolutional layers 511 is that spatially local correlation of the input data can exploited by enforcing a local connectivity pattern between nodes of adjacent layers, in particular by each node being connected to only a small region of the nodes of the preceding layer.

A pooling layer 513 is a connection layer between an anterior node layer 512 (with node values x(n-1)) and a posterior node layer 514 (with node values x(n)). In particular, a pooling layer 513 can be characterized by the structure and the weights of the edges and the activation function forming a pooling operation based on a non-linear pooling function f. For example, in the two-dimensional case the values x(n) of the nodes 524 of the posterior node layer 514 can be calculated based on the values x(n-1) of the nodes 522 of the anterior node layer 512 as

$$x^{(n)_b}[i,j] = f(x^{(n-1)}[id_1, jd_2], ..., x^{(n-1)_b}[(i+1)d_1\text{-}1, (j+1)d_2\text{-}1])$$

In other words, by using a pooling layer 513 the number of nodes 522, 524 can be reduced, by re-placing a number d1·d2 of neighboring nodes 522 in the anterior node layer 512 with a single node 522 in the posterior node layer 514 being calculated as a function of the values of said number of neighboring nodes. In particular, the pooling function f can be the max-function, the average or the L2-Norm. In particular, for a pooling layer 513 the weights of the incoming edges are fixed and are not modified by training.

The advantage of using a pooling layer 513 is that the number of nodes 522, 524 and the number of parameters is reduced. This leads to the amount of computation in the network being reduced and to a control of overfitting.

In the displayed embodiment, the pooling layer 513 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 500 are fully connected layers 515. A fully connected layer 515 is a connection layer between an anterior node layer 514 and a posterior node layer 516. A fully connected layer 513 can be characterized by the fact that a majority, in particular, all edges between nodes 514 of the anterior node layer 514 and the nodes 516 of the posterior node layer are present, and wherein the weight of each of these edges can be adjusted individually.

In this embodiment, the nodes 524 of the anterior node layer 514 of the fully connected layer 515 are displayed both as two-dimensional matrices, and additionally as non-related nodes (indicated as a line of nodes, wherein the number of nodes was reduced for a better presentability). This operation is also denoted as "flattening". In this embodiment, the number of nodes 526 in the posterior node layer 516 of the fully connected layer 515 smaller than the number of nodes 524 in the anterior node layer 514. Alternatively, the number of nodes 526 can be equal or larger.

Furthermore, in this embodiment the Softmax activation function is used within the fully connected layer 515. By applying the Softmax function, the sum the values of all nodes 526 of the output layer 516 is 1, and all values of all nodes 526 of the output layer 516 are real numbers between 0 and 1. In particular, if using the convolutional neural network 500 for categorizing input data, the values of the output layer 516 can be interpreted as the probability of the input data falling into one of the different categories.

In particular, convolutional neural networks 500 can be trained based on the backpropagation algorithm. For preventing overfitting, methods of regularization can be used, e.g., dropout of nodes 520, ..., 524, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

A generative adversarial model (an acronym is GA model) comprises a generative function and a discriminative function, wherein the generative function creates synthetic data, and the discriminative function distinguishes between synthetic and real data. By training the generative function and/or the discriminative function on the one hand the generative function is configured to create synthetic data which is incorrectly classified by the discriminative function as real, on the other hand the discriminative function is configured to distinguish between real data and synthetic data generated by the generative function. In the notion of game theory, a generative adversarial model can be interpreted as a zero-sum game. The training of the generative function and/or of the discriminative function is based, in particular, on the minimization of a cost function.

By using a GA model, based on a set of training data synthetic data can be generated that has the same characteristics as the training data set. The training of the GA model can be based on data not being annotated (unsupervised learning), so that there is low effort in training a GA model.

Figure 6 shows a data flow diagram according to an embodiment for using a generative adversarial network for creating synthetic output data G(x) 608 based on input data x 602 that is indistinguishable from real output data y 604, in accordance with one or more embodiments. The synthetic output data G(x) 608 has the same structure as the real output data y 604, but its content is not derived from real world data.

The generative adversarial network comprises a generator function G 606 and a classifier function C 610 which are trained jointly. The task of the generator function G 606 is to provide realistic synthetic output data G(x) 608 based on input data x 602, and the task of the classifier function C 610 is to distinguish between real output data y 604 and synthetic output data G(x) 608. In particular, the output of the classifier function C 610 is a real number between 0 and 1 corresponding to the probability of the input value being real data, so that an ideal classifier function would calculate an output value of $C(y)$ 614 $\approx$ 1 for real data y 604 and $C(G(x))$ 612 $\approx$ 0 for synthetic data G(x) 608.

Within the training process, parameters of the generator function G 606 are adapted so that the synthetic output data G(x) 608 has the same characteristics as real output data y 604, so that the classifier function C 610 cannot distinguish between real and synthetic data anymore. At the same time, parameters of the classifier function C 610 are adapted so that it distinguishes between real and synthetic data in the best possible way. Here, the training relies on pairs comprising input data x 602 and the corresponding real output data y 604. Within a single training step, the generator function G 606 is applied to the input data x 602 for generating synthetic output data G(x) 608. Furthermore, the classifier function C 610 is applied to the real output data y 604 for generating a first classification result $C(y)$ 614. Additionally, the classifier function C 610 is applied to the synthetic output data G(x) 608 for generating a second classification result $C(G(x))$ 612.

Adapting the parameters of the generative function G 606 and the classifier function C 610 is based on minimizing a cost function by using the backpropagation algorithm, respectively. In this embodiment, the cost function $K_C$ for the classifier function C 610 is $K_C \propto - BCE(C(y), 1) - BCE(C(G(x)), 0)$, wherein BCE denotes the binary cross entropy defined as $BCE(z, z') = z' \cdot log(z) + (1 - z') \cdot log(1 - z)$. By using this cost function, both wrongly classifying real output data as synthetic (indicated by $C(y) \approx 0$) and wrongly classifying synthetic output data as real (indicated as $C(G(x))$ 612 $\approx$ 1) increases the cost function $K_C$ to be minimized. Furthermore, the cost function $K_G$ for the generator function G 606 is $K_G \propto - BCE(C(G(x)), 1) = - log(C(G(x)))$. By using this cost function, correctly classified synthetic output data (indicated as $C(G(x))$ 612 $\approx$ 0) leads to an increase of the cost function $K_G$ to be minimized.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

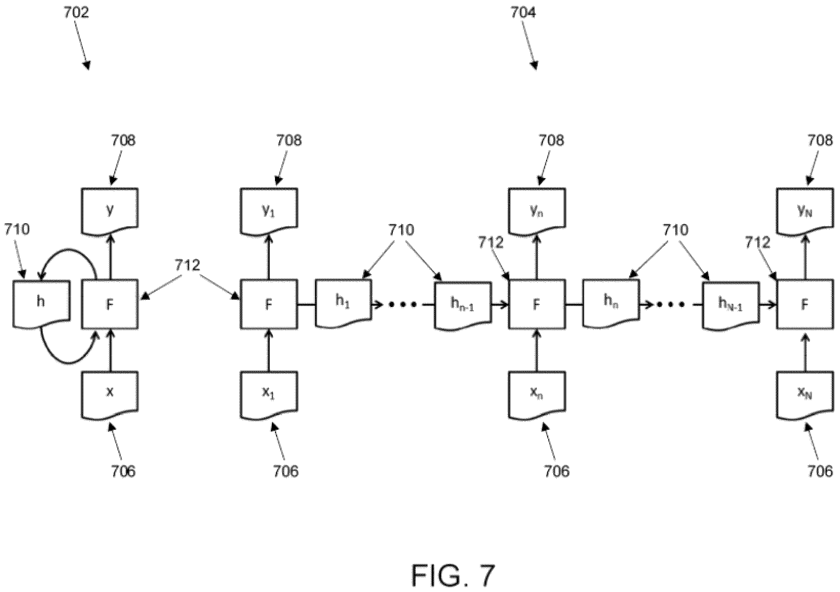

Fig. 7 shows the schematic structure of a recurrent machine learning model F, both in a recurrent representation 702 and in an unfolded representation 704, that may be used to implement one or more machine learning models described herein. The recurrent machine learning model takes as input several input datasets $x, x_1, ..., x_N$ 706 and creates a corresponding set of output datasets $y, y_1, ..., y_N$ 708. Furthermore, the output depends on a so-called hidden vector $h, h_1, ..., h_N$ 710, which implicitly comprises information about input datasets previously used as input for the recurrent machine learning model F 712. By using these hidden vectors $h, h_1, ..., h_N$ 710, a sequentiality of the input datasets can be leveraged.

In a single step of the processing, the recurrent machine learning model F 712 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 712 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 712 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(x_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-3. Certain steps or functions of the methods and workflows described herein, including one or more of the steps or functions of Figures 1-3, may be performed by a server or by another processor in a network-based cloud-computing system. Certain steps or functions of the methods and workflows described herein, including one or more of the steps of Figures 1-3, may be performed by a client computer in a network-based cloud computing system. The steps or functions of the methods and workflows described herein, including one or more of the steps of Figures 1-3, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-3, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 802 that may be used to implement systems, apparatuses, and methods described herein is depicted in Figure 8. Computer 802 includes a processor 804 operatively coupled to a data storage device 812 and a memory 810. Processor 804 controls the overall operation of computer 802 by executing computer program instructions that define such operations. The computer program instructions may be stored in data storage device 812, or other computer readable medium, and loaded into memory 810 when execution of the computer program instructions is desired. Thus, the method and workflow steps or functions of Figures 1-3 can be defined by the computer program instructions stored in memory 810 and/or data storage device 812 and controlled by processor 804 executing the computer program instructions. For example, the computer program instructions can be implemented as computer executable code programmed by one skilled in the art to perform the method and workflow steps or functions of Figures 1-3. Accordingly, by executing the computer program instructions, the processor 804 executes the method and workflow steps or functions of Figures 1-3. Computer 802 may also include one or more network interfaces 806 for communicating with other devices via a network. Computer 802 may also include one or more input/output devices 808 that enable user interaction with computer 802 (e.g., display, keyboard, mouse, speakers, buttons, etc.).

Processor 804 may include both general and special purpose microprocessors, and may be the sole processor or one of multiple processors of computer 802. Processor 804 may include one or more central processing units (CPUs), for example. Processor 804, data storage device 812, and/or memory 810 may include, be supplemented by, or incorporated in, one or more application-specific integrated circuits (ASICs) and/or one or more field programmable gate arrays (FPGAs).

Data storage device 812 and memory 810 each include a tangible non-transitory computer readable storage medium. Data storage device 812, and memory 810, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 808 may include peripherals, such as a printer, scanner, display screen, etc. For example, input/output devices 808 may include a display device such as a cathode ray tube (CRT) or liquid crystal display (LCD) monitor for displaying information to the user, a keyboard, and a pointing device such as a mouse or a trackball by which the user can provide input to computer 802.

An image acquisition device 814 can be connected to the computer 802 to input image data (e.g., medical images) to the computer 802. It is possible to implement the image acquisition device 814 and the computer 802 as one device. It is also possible that the image acquisition device 814 and the computer 802 communicate wirelessly through a network. In a possible embodiment, the computer 802 can be located remotely with respect to the image acquisition device 814.

Any or all of the systems, apparatuses, and methods discussed herein may be implemented using one or more computers such as computer 802.

One skilled in the art will recognize that an implementation of an actual computer or computer system may have other structures and may contain other components as well, and that Figure 8 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

The following is a list of non-limiting illustrative embodiments disclosed herein:

Illustrative embodiment 1. A computer-implemented method comprising: receiving one or more input medical images of an anatomical object of a patient; extracting characteristics of the anatomical object from the one or more input medical images; determining a risk associated with a medical procedure to be performed on the anatomical object based on the one or more input medical images and the extracted characteristics of the anatomical object using one or more machine learning based risk assessment models; performing a simulation of the medical procedure on the anatomical object based on the one or more input medical images, the extracted characteristics of the anatomical object, and the risk associated with the medical procedure; automatically making one or more clinical decisions associated with the medical procedure based on the risk associated with the medical procedure and results of the simulation; and outputting the one or more clinical decisions.

Illustrative embodiment 2. The computer-implemented method of illustrative embodiment 1, wherein the one or more clinical decisions comprises a decision to perform the medical procedure, the method further comprising: receiving one or more preoperative medical images of the anatomical object of the patient for performing the medical procedure; extracting additional characteristics of the anatomical object from the one or more preoperative medical images; determining an updated risk associated with the medical procedure to be performed on the anatomical object based on the one or more preoperative medical images and the extracted additional characteristics of the anatomical object using the one or more machine learning based risk assessment models; performing an additional simulation of the medical procedure on the anatomical object based on the one or more preoperative medical images, the extracted additional characteristics of the anatomical object, and the updated risk associated with the medical procedure; automatically making one or more additional clinical decisions associated with the medical procedure based on the updated risk associated with the medical procedure and results of the additional simulation; and outputting the one or more additional clinical decisions.

Illustrative embodiment 3. The computer-implemented method of illustrative embodiment 2, further comprising: performing the medical procedure based on the one or more additional clinical decisions.

Illustrative embodiment 4. The computer-implemented method of illustrative embodiment 3, further comprising: determining a post-procedure risk associated with the performed medical procedure.

Illustrative embodiment 5. The computer-implemented method of any of illustrative embodiments 1-4, further comprising: iteratively repeating the steps of 1) determining the risk associated with the medical procedure based on the results of the simulation and 2) performing the simulation of the medical procedure to optimize a cost function.

Illustrative embodiment 6. The computer-implemented method of any of illustrative embodiments 1-5, wherein the anatomical object comprises a stenosis and extracting characteristics of the anatomical object from the one or more input medical images comprises: extracting geometry characteristics associated with the stenosis and plaque characteristics associated with the stenosis from the one or more input medical images.

Illustrative embodiment 7. The computer-implemented method of any of illustrative embodiments 1-6, further comprising receiving characteristics of the medical procedure, wherein performing a simulation of the medical procedure on the anatomical object based on the one or more input medical images, the extracted characteristics of the anatomical object, and the risk associated with the medical procedure comprises: performing the simulation of the medical procedure on the anatomical object further based on the characteristics of the medical procedure.

Illustrative embodiment 8. The computer-implemented method of any of illustrative embodiments 1-7, wherein the anatomical object comprises a stenosis, the medical procedure comprises a PCI (percutaneous coronary intervention) procedure to place a stent at the stenosis, and the risk comprises a risk of under-expansion of the stent.

Illustrative embodiment 9. The computer-implemented method of any of illustrative embodiments 1-8, wherein the one or more input medical images comprises at least one of a photon counting computed tomography image, an x-ray angiography image, an intravascular ultrasound image, or an optical coherence tomography image.

Illustrative embodiment 10. An apparatus comprising: means for receiving one or more input medical images of an anatomical object of a patient; means for extracting characteristics of the anatomical object from the one or more input medical images; means for determining a risk associated with a medical procedure to be performed on the anatomical object based on the one or more input medical images and the extracted characteristics of the anatomical object using one or more machine learning based risk assessment models; means for performing a simulation of the medical procedure on the anatomical object based on the one or more input medical images, the extracted characteristics of the anatomical object, and the risk associated with the medical procedure; means for automatically making one or more clinical decisions associated with the medical procedure based on the risk associated with the medical procedure and results of the simulation; and means for outputting the one or more clinical decisions.

Illustrative embodiment 11. The apparatus of illustrative embodiment 10, wherein the one or more clinical decisions comprises a decision to perform the medical procedure, the apparatus further comprising: means for receiving one or more preoperative medical images of the anatomical object of the patient for performing the medical procedure; means for extracting additional characteristics of the anatomical object from the one or more preoperative medical images; means for determining an updated risk associated with the medical procedure to be performed on the anatomical object based on the one or more preoperative medical images and the extracted additional characteristics of the anatomical object using the one or more machine learning based risk assessment models; means for performing an additional simulation of the medical procedure on the anatomical object based on the one or more preoperative medical images, the extracted additional characteristics of the anatomical object, and the updated risk associated with the medical procedure;

means for automatically making one or more additional clinical decisions associated with the medical procedure based on the updated risk associated with the medical procedure and results of the additional simulation; and means for outputting the one or more additional clinical decisions.

Illustrative embodiment 12. The apparatus of any of illustrative embodiments 10-11, further comprising: means for performing the medical procedure based on the one or more additional clinical decisions.

Illustrative embodiment 13. The apparatus of any of illustrative embodiments 10-12, further comprising: means for determining a post-procedure risk associated with the performed medical procedure.

Illustrative embodiment 14. The apparatus of any of illustrative embodiments 10-13, further comprising: means for iteratively repeating the steps of 1) determining the risk associated with the medical procedure based on the results of the simulation and 2) performing the simulation of the medical procedure to optimize a cost function.

Illustrative embodiment 15. A non-transitory computer-readable storage medium comprising instructions which, when executed by a computer, cause the computer to carry out operations comprising: receiving one or more input medical images of an anatomical object of a patient; extracting characteristics of the anatomical object from the one or more input medical images; determining a risk associated with a medical procedure to be performed on the anatomical object based on the one or more input medical images and the extracted characteristics of the anatomical object using one or more machine learning based risk assessment models; performing a simulation of the medical procedure on the anatomical object based on the one or more input medical images, the extracted characteristics of the anatomical object, and the risk associated with the medical procedure; automatically making one or more clinical decisions associated with the medical procedure based on the risk associated with the medical procedure and results of the simulation; and outputting the one or more clinical decisions.

Illustrative embodiment 16. The non-transitory computer-readable storage medium of illustrative embodiment 15, wherein the one or more clinical decisions comprises a decision to perform the medical procedure, the operations further comprising: receiving one or more preoperative medical images of the anatomical object of the patient for performing the medical procedure; extracting additional characteristics of the anatomical object from the one or more preoperative medical images; determining an updated risk associated with the medical procedure to be performed on the anatomical object based on the one or more preoperative medical images and the extracted additional characteristics of the anatomical object using the one or more machine learning based risk assessment models; performing an additional simulation of the medical procedure on the anatomical object based on the one or more preoperative medical images, the extracted additional characteristics of the anatomical object, and the updated risk associated with the medical procedure; automatically making one or more additional clinical decisions associated with the medical procedure based on the updated risk associated with the medical procedure and results of the additional simulation; and outputting the one or more additional clinical decisions.

Illustrative embodiment 17. The non-transitory computer-readable storage medium of any of illustrative embodiments 15-16, wherein the anatomical object comprises a stenosis and extracting characteristics of the anatomical object from the one or more input medical images comprises: extracting geometry characteristics associated with the stenosis and plaque characteristics associated with the stenosis from the one or more input medical images.

Illustrative embodiment 18. The non-transitory computer-readable storage medium of any of illustrative embodiments 15-17, further comprising receiving characteristics of the medical procedure, wherein performing a simulation of the medical procedure on the anatomical object based on the one or more input medical images, the extracted characteristics of the anatomical object, and the risk associated with the medical procedure comprises: performing the simulation of the medical procedure on the anatomical object further based on the characteristics of the medical procedure.

Illustrative embodiment 19. The non-transitory computer-readable storage medium of any of illustrative embodiments 15-18, wherein the anatomical object comprises a stenosis, the medical procedure comprises a PCI (percutaneous coronary intervention) procedure to place a stent at the stenosis, and the risk comprises a risk of under-expansion of the stent.

Illustrative embodiment 20. The non-transitory computer-readable storage medium of any of illustrative embodiments 15-19, wherein the one or more input medical images comprises at least one of a photon counting computed tomography image, an x-ray angiography image, an intravascular ultrasound image, or an optical coherence tomography image.

**Claims**

**1.** A computer-implemented method comprising:

receiving (106, 202) one or more input medical images of an anatomical object of a patient;
extracting (106, 204) characteristics of the anatomical object from the one or more input medical images;
determining (108, 206) a risk associated with a medical procedure to be performed on the anatomical object based on the one or more input medical images and the extracted characteristics of the anatomical object using one or more machine learning based risk assessment models;

performing (110, 208) a simulation of the medical procedure on the anatomical object based on the one or more input medical images, the extracted characteristics of the anatomical object, and the risk associated with the medical procedure;

automatically making (112, 210) one or more clinical decisions associated with the medical procedure based on the risk associated with the medical procedure and results of the simulation; and

outputting (212) the one or more clinical decisions.

2. The computer-implemented method of claim 1, wherein the one or more clinical decisions comprises a decision to perform the medical procedure, the method further comprising:

receiving (118, 302) one or more preoperative medical images of the anatomical object of the patient for performing the medical procedure;

extracting (304) additional characteristics of the anatomical object from the one or more preoperative medical images;

determining (120, 306) an updated risk associated with the medical procedure to be performed on the anatomical object based on the one or more preoperative medical images and the extracted additional characteristics of the anatomical object using the one or more machine learning based risk assessment models;

performing (122, 308) an additional simulation of the medical procedure on the anatomical object based on the one or more preoperative medical images, the extracted additional characteristics of the anatomical object, and the updated risk associated with the medical procedure;

automatically making (124, 310) one or more additional clinical decisions associated with the medical procedure based on the updated risk associated with the medical procedure and results of the additional simulation; and

outputting (312) the one or more additional clinical decisions.

3. The computer-implemented method of claim 2, further comprising:
performing the medical procedure based on the one or more additional clinical decisions.

4. The computer-implemented method of claim 3, further comprising:
determining (126) a post-procedure risk associated with the performed medical procedure.

5. The computer-implemented method of claim 1, further comprising:
iteratively repeating the steps of 1) determining the risk associated with the medical procedure based on the results of the simulation and 2) performing the simulation of the medical procedure to optimize a cost function.

6. The computer-implemented method of claim 1, wherein the anatomical object comprises a stenosis and extracting characteristics of the anatomical object from the one or more input medical images comprises:
extracting geometry characteristics associated with the stenosis and plaque characteristics associated with the stenosis from the one or more input medical images.

7. The computer-implemented method of claim 1, further comprising receiving characteristics of the medical procedure, wherein performing a simulation of the medical procedure on the anatomical object based on the one or more input medical images, the extracted characteristics of the anatomical object, and the risk associated with the medical procedure comprises:
performing the simulation of the medical procedure on the anatomical object further based on the characteristics of the medical procedure.

8. The computer-implemented method of claim 1, wherein the anatomical object comprises a stenosis, the medical procedure comprises a PCI (percutaneous coronary intervention) procedure to place a stent at the stenosis, and the risk comprises a risk of under-expansion of the stent.

9. The computer-implemented method of claim 1, wherein the one or more input medical images comprises at least one of a photon counting computed tomography image, an x-ray angiography image, an intravascular ultrasound image, or an optical coherence tomography image.

10. An apparatus comprising:

means for receiving (106, 202) one or more input medical images of an anatomical object of a patient;

means for extracting (106, 204) characteristics of the anatomical object from the one or more input medical images;

means for determining (108, 206) a risk associated with a medical procedure to be performed on the anatomical object based on the one or more input medical images and the extracted characteristics of the anatomical object using one or more machine learning based risk assessment models;

means for performing (110, 208) a simulation of the medical procedure on the anatomical object based on the one or more input medical images, the extracted characteristics of the anatomical object, and the risk associated with the medical procedure;

means for automatically making (112, 210) one or more clinical decisions associated with the medical procedure based on the risk associated with the medical procedure and results of the simulation; and

means for outputting (212) the one or more clinical decisions.

11. The apparatus of claim 10, wherein the one or more clinical decisions comprises a decision to perform the medical procedure, the apparatus further comprising:

means for receiving (118, 302) one or more preoperative medical images of the anatomical object of the patient for performing the medical procedure;

means for extracting (304) additional characteristics of the anatomical object from the one or more preoperative medical images;

means for determining (120, 306) an updated risk associated with the medical procedure to be performed on the anatomical object based on the one or more preoperative medical images and the extracted additional characteristics of the anatomical object using the one or more machine learning based risk assessment models;

means for performing (122, 308) an additional simulation of the medical procedure on the anatomical object based on the one or more preoperative medical images, the extracted additional characteristics of the anatomical object, and the updated risk associated with the medical procedure;

means for automatically making (124, 310) one or more additional clinical decisions associated with the medical procedure based on the updated risk associated with the medical procedure and results of the additional simulation; and

means for outputting (312) the one or more additional clinical decisions.

12. The apparatus of claim 11, further comprising:
means for performing the medical procedure based on the one or more additional clinical decisions.

13. The apparatus of claim 12, further comprising:
means for determining (126) a post-procedure risk associated with the performed medical procedure.

14. The apparatus of claim 10, further comprising:
means for iteratively repeating the steps of 1) determining the risk associated with the medical procedure based on the results of the simulation and 2) performing the simulation of the medical procedure to optimize a cost function.

15. A non-transitory computer-readable storage medium comprising instructions which, when executed by a computer, cause the computer to carry out operations comprising:

receiving (106, 202) one or more input medical images of an anatomical object of a patient;

extracting (106, 204) characteristics of the anatomical object from the one or more input medical images;

determining (108, 206) a risk associated with a medical procedure to be performed on the anatomical object based on the one or more input medical images and the extracted characteristics of the anatomical object using one or more machine learning based risk assessment models;

performing (110, 208) a simulation of the medical procedure on the anatomical object based on the one or more input medical images, the extracted characteristics of the anatomical object, and the risk associated with the medical procedure;

automatically making (112, 210) one or more clinical decisions associated with the medical procedure based on the risk associated with the medical procedure and results of the simulation; and

outputting (212) the one or more clinical decisions.

16. The non-transitory computer-readable storage medium of claim 15, wherein the one or more clinical decisions comprises a decision to perform the medical procedure, the operations further comprising:

receiving (118, 302) one or more preoperative medical images of the anatomical object of the patient for performing the medical procedure;

extracting (304) additional characteristics of the anatomical object from the one or more preoperative medical images;

determining (120, 306) an updated risk associated with the medical procedure to be performed on the anatomical object based on the one or more preoperative medical images and the extracted additional characteristics of the anatomical object using the one or more machine learning based risk assessment models;

performing (122, 308) an additional simulation of the medical procedure on the anatomical object based on the one or more preoperative medical images, the extracted additional characteristics of the anatomical object, and the updated risk associated with the medical procedure;

automatically making (124, 310) one or more additional clinical decisions associated with the medical procedure based on the updated risk associated with the medical procedure and results of the additional simulation; and

outputting (312) the one or more additional clinical decisions.

17. The non-transitory computer-readable storage medium of claim 15, wherein the anatomical object comprises a stenosis and extracting characteristics of the anatomical object from the one or more input medical images comprises: extracting geometry characteristics associated with the stenosis and plaque characteristics associated with the stenosis from the one or more input medical images.

18. The non-transitory computer-readable storage medium of claim 15, further comprising receiving characteristics of the medical procedure, wherein performing a simulation of the medical procedure on the anatomical object based on the one or more input medical images, the extracted characteristics of the anatomical object, and the risk associated with the medical procedure comprises: performing the simulation of the medical procedure on the anatomical object further based on the characteristics of the medical procedure.

19. The non-transitory computer-readable storage medium of claim 15, wherein the anatomical object comprises a stenosis, the medical procedure comprises a PCI (percutaneous coronary intervention) procedure to place a stent at the stenosis, and the risk comprises a risk of under-expansion of the stent.

20. The non-transitory computer-readable storage medium of claim 15, wherein the one or more input medical images comprises at least one of a photon counting computed tomography image, an x-ray angiography image, an intravascular ultrasound image, or an optical coherence tomography image.

# FIG. 1

# FIG. 2

200

Receive one or more input medical images of an anatomical object of a patient
202

Extract characteristics of the anatomical object from the one or more input medical images
204

Determine a risk associated with a medical procedure to be performed on the anatomical object based on the one or more input medical images and the extracted characteristics of the anatomical object
206

Perform a simulation of the medical procedure on the anatomical object based on the one or more input medical images, the extracted characteristics of the anatomical object, and the risk associated with the medical procedure
208

Automatically make one or more clinical decisions associated with the medical procedure based on the risk associated with the medical procedure and results of the simulation
210

Output the one or more clinical decisions
212

# FIG. 3

300

Receive one or more preoperative medical images of the anatomical object of the patient for performing the medical procedure
302

Extract additional characteristics of the anatomical object from the one or more preoperative medical images
304

Determine an updated risk associated with the medical procedure to be performed on the anatomical object based on the one or more preoperative medical images and the extracted additional characteristics of the anatomical object
306

Perform an additional simulation of the medical procedure on the anatomical object based on the one or more preoperative medical images, the extracted additional characteristics of the anatomical object, and the updated risk associated with the medical procedure
308

Automatically make one or more additional clinical decisions associated with the medical procedure based on the updated risk associated with the medical procedure and results of the additional simulation
310

Output the one or more additional clinical decisions
312

FIG. 4

FIG. 5

FIG. 6

FIG. 7

FIG. 8

802

Network interface
806

I/O
808

Processor
804

Storage
812

Memory
810

Image Acquisition Device
814

Europäisches
Patentamt
European
Patent Office
Office européen
des brevets

## EUROPEAN SEARCH REPORT

**Application Number**

EP 24 46 5518

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/025270 A1 (SIEMENS HEALTHCARE GMBH [DE]) 7 February 2019 (2019-02-07) * The whole document, in particular: Paragraphs [0007], [0045] - [0051], [0070] - [0084], [0091], [0092]; Figures 3, 6, 10. * | 1-20 | INV. G16H20/40 G16H50/30 G16H30/40 G16H50/20 G16H50/50 G16H50/70 |
| X | US 2024/046476 A1 (AMIS GREGORY PATRICK [US] ET AL) 8 February 2024 (2024-02-08) * The whole document, in particular: Paragraphs [0004] - [0012], [0041], [0042], [0054], [0060], [0077], [0090] - [0098], [0112], [0113], [0123], [0130]. * | 1-20 | |
| X | WO 2024/058835 A1 (MEDTRONIC VASCULAR INC [US]) 21 March 2024 (2024-03-21) * The whole document, in particular: Paragraphs [0016] - [0075], [0251] - [0269]; Figure 23. * | 1-20 | |

**TECHNICAL FIELDS SEARCHED (IPC)**

G16H

The present search report has been drawn up for all claims

| Place of search | Date of completion of the search | Examiner |
|---|---|---|
| Munich | 13 September 2024 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 46 5518

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2024

| Patent document cited in search report | Publication date | Patent family member(s) | | Publication date |
|---|---|---|---|---|
| WO 2019025270 A1 | 07-02-2019 | NONE | | |
| US 2024046476 A1 | 08-02-2024 | CA | 3146613 A1 | 11-02-2021 |
| | | CN | 114424290 A | 29-04-2022 |
| | | CN | 116884580 A | 13-10-2023 |
| | | EP | 4010874 A1 | 15-06-2022 |
| | | JP | 2022543643 A | 13-10-2022 |
| | | US | 2021042927 A1 | 11-02-2021 |
| | | US | 2022335616 A1 | 20-10-2022 |
| | | US | 2024046476 A1 | 08-02-2024 |
| | | WO | 2021026224 A1 | 11-02-2021 |
| WO 2024058835 A1 | 21-03-2024 | NONE | | |

EPO FORM P0459

For more details about this annex : see Official Journal of the European Patent Office, No. 12/82